# EUROPÄISCHE PATENTSCHRIFT

(11) **EP 1 915 974 B1**
(45) Veröffentlichungstag und Bekanntmachung des Hinweises auf die Patenterteilung: **08.07.2009**
(21) Anmeldenummer: 07019015.2
(22) Anmeldetag: 27.09.2007
(51) Int. Cl.: A61F 2/38

(54) **Endoprothese, insbesondere Kniegelenkprothese**
Endoprosthesis, in particular knee joint prosthesis
Endoprothèse, en particulier prothèse d'articulation du pied

(30) Priorität: 27.10.2006 DE 102006051393
(43) Veröffentlichungstag der Anmeldung: 30.04.2008
(73) Patentinhaber: Krehl, Frieder, W. Dr., 78048 Villingen-Schwenningen (DE)
(72) Erfinder: Krehl, Frieder, W. Dr., 78048 Villingen-Schwenningen (DE)
(74) Vertreter: Patentanwälte Westphal, Mussgnug & Partner

(56) Entgegenhaltungen:
- EP-A- 1 537 839
- WO-A-96/19162
- WO-A-20/05025451
- US-A1- 2005 192 674

## Beschreibung

Die Erfindung bezieht sich auf eine Gelenkendoprothese der im Oberbegriff des Patentanspruchs 1 genannten Art.

Derartige Gelenkendoprothesen, sogenannte künstliche Gelenke, sind dazu bestimmt, ein geschädigtes oder erkranktes Gelenk, z.B. ein Kniegelenk, zu ersetzen.

Sie bestehen im Falle des Kniegelenkes aus metallischen Gelenkköpfen, welche operativ mit der Tibia und dem Femur zu verbinden sind. Zwischen den Gelenkköpfen ist ein Inlay z.B. aus Polyäthylen angeordnet, welches den direkten Metallkontakt verhindert und teilweise die Inkongruenz der Ober- und Unterteil bildenden Gelenkköpfe ausgleicht.

Wegen der erheblichen im Wesentlichen punktuellen Dauerbelastung ist die Ausbildung dieses Inlays nicht unproblematisch. So ist Kaltfluß, Delamination und Abrieb des Inlaymaterials die Folge.

Mit der in WO 99/42061 veröffentlichten Erfindung wurde bereits ein Inlay vorgeschlagen, das aus einem nachgiebigen Polster, einer auf dem Polster aufliegenden flexiblen Membran und auf der Membran angeordneten Stützkörpern besteht, deren Oberfläche der Lagerung der Gelenkköpfe dient. Ein derartiges Inlay reduziert die Reibungskraft und vermindert den Abrieb und dessen Einlagerung in das das Gelenk umgebende Gewebe, was zu Störungen und Schädigung führen kann.

Nicht gelöst ist bei dieser Gestaltung des Inlays die Verbindung der Stützkörper mit der Membran. Zum andern haben Untersuchungen ergeben, dass Klebeverbindungen den bei der Abrollbewegung der Gelenkköpfe auftretenden Scherkräften auf Dauer nicht standhalten.

So liegt der vorliegenden Erfindung die Aufgabe zugrunde, eine Gelenkendoprothese der im Oberbegriff des Patentanspruchs 1 genannten Art zu schaffen, deren Stützkörper dauerhaft fixiert sind, ohne die Flexibilität zu beeinträchtigen.

Gelöst wird diese Aufgabe nach vorliegender Erfindung durch eine zweite Membran, die oberhalb und parallel zu der erstgenannten Membran verläuft und Halteöffnungen aufweist, in welchen die Stützkörper verankert sind und sich seitlich aneinander und mit ihrer Unterseite auf der ersten Membran abstützen, wobei beide Membranen am Grundkörper befestigt sind.

Hierbei sitzen die Stützkörper auf der ersten Membran auf und sind mit den benachbarten Stützkörpern ausschließlich über die zweite Membran verbunden. Damit ist eine eingeschränkte Beweglichkeit der Stützkörper senkrecht zur Membran möglich, wobei sich die Stützkörper seitlich an den benachbarten abstützen. Nach dem Vorschlag gemäß Anspruch 5 können sich die am Rande gelegenen Stützkörper an den seitlichen Wänden der als Grundkörper dienenden Wanne abstützen; welche die entstehenden Scherkräfte aufnehmen, so dass die zweite der Halterung der Stützkörper dienende Membran nicht auf Scherung beansprucht wird.

Die spezielle Ausbildung der Stützkörper ist Gegenstand der Unteransprüche 2 bis 4.

Vorschläge zur Befestigung der Membran am Grundkörper, zur Materialwahl der Membranen und der Stützkörper sowie zur Gestaltung des Polsters sind mit den Ansprüchen 6 bis 8 angegeben.

Als Material für die Membran hat sich wegen seiner großen Flexibilität insbesondere Silikon bewährt.

Die erfindungsgemäße Gestaltung ist nach dem Vorschlag gemäß Anspruch 9 besonders für Kniegelenkprothesen geeignet.

Der Gegenstand der Erfindung ist nachstehend anhand eines in der Zeichnung dargestellten Ausführungsbeispiels im Einzelnen erläutert. In den Zeichnungen zeigen:
- Figur 1: Querschnitt eines erfindungsgemäßen Inlays für eine Kniegelenkprothese,
- Figur 2: vergrößerte Teilaufsicht auf das Inlay gemäß Figur 1 und
- Figur 3: vergrößerter Querschnitt eines Stützkörpers.

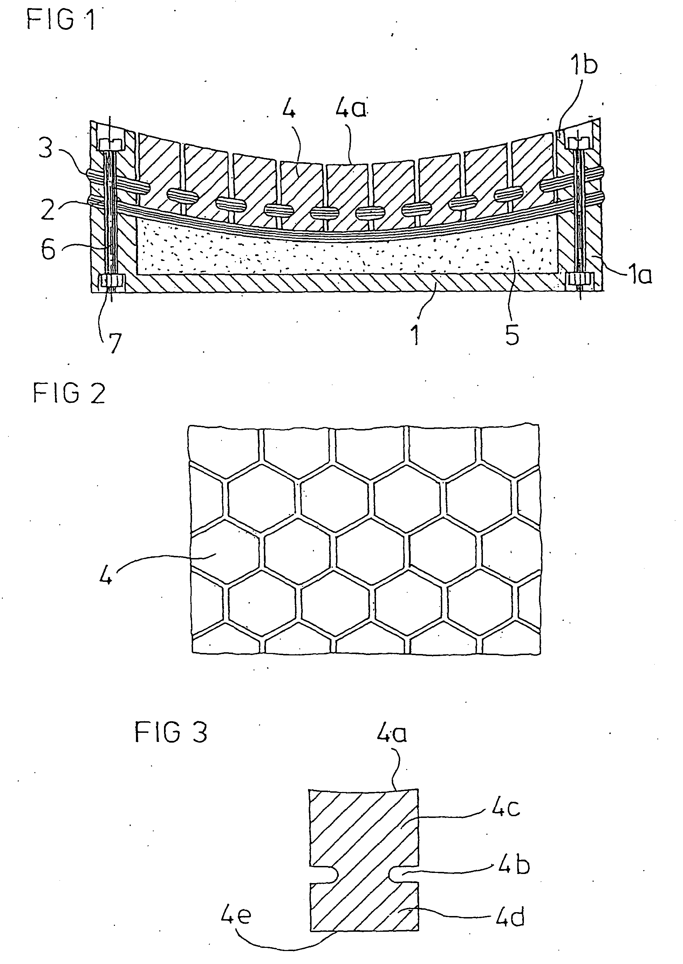

In Figur 1 ist ein erfindungsgemäß gestaltetes Inlay dargestellt, das bestimmungsgemäß zwischen den in der Regel aus Stahl bestehenden Gelenkköpfen der Gelenkprothese angeordnet ist, welche ihrerseits mit Schrauben, Dornen oder mit Zement im Knochen, im Falle einer Kniegelenkprothese mit dem Tibia- bzw. Femurknochen, verbunden sind.

Das Inlay besteht im Wesentlichen aus einem flexiblen, aber nicht kompressiblen Polster 5, auf welchem eine erste Membran 2 und darüber eine zweite Membran 3 mit in dieser verankerten Stützkörpern 4 angeordnet sind. Die Membranen 2 und 3 bestehen aus Silikon, die Stützkörper 4 aus Polyäthylen. Das Polster 5 ist in einem als Wanne ausgebildeten, gleichfalls aus Polyäthylen bestehenden Grundkörper 1 angeordnet, deren seitliche Wände 1a bis zur Oberfläche 4a der Stützkörper 4 hochgezogen sind. An diesen Wänden 1a sind die Membranen 2 und 3 mit Hilfe von Schrauben 6 und Muttern 7 festgelegt.

Die Oberflächen 4a der Stützkörper 4 und 1b der Wannenwände 1a bilden eine leicht konkave Lagerfläche für den nicht dargestellten Gelenkkopf mit seiner konvexen Oberfläche.

Wie vor allem Figur 3 erkennen lässt, sind die im Querschnitt doppel-T-förmig ausgebildeten Stützkörper 4 durch eine Einschnürung 4b in ein Oberteil 4c und ein Fußteil 4d unterteilt. Sie sind mit ihrem Fußteil 4d in entsprechende Halteöffnungen der Membran 2 eingeklipst und damit festgelegt. Die Unterseite 4e des Fußteiles stützt sich auf der Oberseite der Membran 2 ab. Seitlich liegen die Stützteile 4 aneinander, wobei die äußeren Stützteile 4 von den seitlichen Wänden 1a der Wanne 1 abgestützt werden.

Wie die Aufsicht gemäß Figur 2 deutlich macht, haben die Stützkörper im Querschnitt die Form eines Sechsecks, wodurch sich eine gute Abstützung nach allen Seiten ergibt.

Durch die erfindungsgemäße Maßnahme sind die Stützkörper 4 in Querrichtung abgestützt, so dass die der Halterung der Stützkörper dienende Membran bei Gelenkbewegungen nicht auf Scherung beansprucht wird.

Die axial wirkenden Druckkräfte werden durch das Polster 5 aufgenommen, das aus einer mit einem nicht oder nur wenig komprimierbaren Fluidum, vorzugsweise einer Kochsalzlösung oder gelartigen Substanz, besteht, welches sich in der nach oben durch die Membran 2 begrenzten Wanne 1 befindet. Dieses Polster 5 sorgt für eine gleichmäßige Verteilung des hydrostatischen Drucks.

Bezugszeichenliste
- 1: Grundkörper, Wanne
- 2: Erste Membran
- 3: Zweite Membran
- 4: Stützkörper
4a Stützkörperoberfläche
4b Einschnürung
4c Oberteil
4d Fußteil
4e Stützkörperunterseite
- 5: Polster

- 6: Schraube
- 7: Mutter

## Patentansprüche

1. Gelenkendoprothese mit Gelenkköpfen zwischen welchen ein Inlay angeordnet ist, das aus einem flexiblen Polster (5), einer auf dem Polster aufliegenden flexiblen ersten Membran (2) und auf der ersten Membran angeordneten Stützkörpern (4) besteht, deren
Oberfläche der Lagerung eines der beiden Gelenkköpfe dient, wobei Polster und Membran von einem Grundkörper (1) aufgenommen sind,
**dadurch gekennzeichnet, dass** oberhalb der ersten Membran (2), parallel zu dieser verlaufend eine Halteöffnungen aufweisende zweite Membran (3) vorgesehen ist, dass die Stützkörper (4) in der zweiten Membran (3) verankert sind, sich seitlich aneinander und mit ihrer Unterseite (4e) auf der ersten Membran (2) abstützen, und dass beide Membranen (2 und 3) am Grundkörper (1) befestigt sind.

2. Gelenkendoprothese nach Anspruch 1,
**dadurch gekennzeichnet, dass** die Stützkörper (4) durch parallel zu den Membranen (2 und 3) verlaufende Einschnürungen (4b) in ein Oberteil (4c) und ein Fußteil (4d) unterteilt sind und mit ihren Einschnürungen (4b) in den Halteöffnungen der zweiten Membran (3) festgelegt sind.

3. Gelenkendoprothese nach Anspruch 2,
**dadurch gekennzeichnet, dass** die Stützkörper (4) im Längsschnitt doppel-T-förmig ausgebildet sind.

4. Gelenkendoprothese nach Anspruch 1, 2 oder 3,
**dadurch gekennzeichnet, dass** die Stützkörper (4) im Querschnitt sechseckförmig sind.

5. Gelenkendoprothese nach einem der Ansprüche 1 bis 4,
**dadurch gekennzeichnet, dass** der Grundkörper als Wanne (1) ausgebildet ist, deren seitliche Wände (1a) bis auf die Höhe der Stützkörperoberfläche (4a) verlängert sind und welche die außen gelegenen Stützkörper (4) seitlich abstützen.

6. Gelenkendoprothese nach einem der Ansprüche 1 bis 5,
**dadurch gekennzeichnet, dass** die Membranen (2, 3) mit dem Grundkörper (1) vorzugsweise mittels die Wände (1a) des Grundkörpers (1) durchsetzenden Schrauben (6, 7), fest verbunden sind.

7. Gelenkendoprothese nach einem der Ansprüche 1 bis 6,
**dadurch gekennzeichnet, dass** die Membranen (2, 3) aus Silikon und die Stützkörper (4) sowie die Wanne (1) aus Polyäthylen bestehen.

8. Gelenkendoprothese nach einem der Ansprüche 1 bis 7,
**dadurch gekennzeichnet, dass** die Wanne (1) zur Bildung des Polsters (5) mit einer Kochsalzlösung oder einer gelartigen Substanz gefüllt und auf der der Lastaufnahme dienenden Seite mit der ersten flexiblen Membran (2) abgedichtet ist.

9. Gelenkendoprothese nach einem der Ansprüche 1 bis 8,
wobei die Gelenkendoprothese eine Kniegelenkprothese ist, bei welcher deren Gelenkköpfe mit der Tibia bzw. dem Femur verbindbar sind.

## Claims

1. A joint endoprosthesis having joint heads between which an inlay is disposed, which inlay consists of a flexible pad (5) , a flexible first membrane (2) resting on the pad, and support elements, (4) disposed on the first membrane, the surface of which is used for supporting one of the two joint heads, the pad and membrane being accommodated by a base body (1),
**characterised in that** above the first membrane (2) and extending parallel thereto there is provided a second membrane (3) is provided which comprises retaining openings,
**in that** the support elements (4) are anchored in the second membrane (3) and laterally support one another and with their undersides (4e') rest on the first membrane (2), **and in that** both membranes (2 and 3) are attached to the base body (1).

2. A joint endoprosthesis according to Claim 1,
**characterised in that** the support elements (4) are divided into an upper part (4c) and a base part (4d) by means of constrictions (4b) running parallel to the membranes (2 and 3), and by their constrictions (4b) they are fixed in the retaining openings in the second membrane (3).

3. A joint endoprosthesis according to Claim 2,
**characterised in that** the support elements (4) have a double T-shaped longitudinal section.

4. A joint endoprosthesis according to claim 1, 2 or 3,
**characterised in that** the support, elements (4) have a hexagonal cross section.

5. A joint endoprosthesis according to one of Claims 1 to 4,
**characterized in that** the base body is designed as a trough (1), the lateral walls (1a) of which extend to the height of the surface (4a) of the support elements and laterally support the exterior support elements (4).

6. A joint endoprosthesis according to one of Claims 1 to 5,
**characterised in that** the membranes (2, 3) are securely connected to the base body (1), preferably by means of screws (6, 7) which pass through the walls (1a) of the base body (1).

7. A joint endoprosthesis according to one of Claims 1 to 6,
**characterised in that** the membranes (2, 3) are made of silicone and the support elements (4) and the trough (1) are made of polyethylene.

8. A joint endoprosthesis according to one of Claims 1 to 7,
**characterised in that** the trough (1) for forming the pad (5) is filled with a saline solution or a gel-like substance, and is sealed by the first flexible membrane (2) on the side serving for load-bearing.

9. A joint endoprosthesis according to one of Claims 1 to 8,
wherein the joint endoprosthesis is a knee joint prosthesis in which the joint heads thereof may be joined to the tibia or the femur.

## Revendications

1. Endoprothèse d'articulation, ayant des têtes d'articulation entre lesquelles est placé un insert (inlay), formé d'un coussin souple (5) d'une première membrane (2) souple appliquée sur le coussin et de premiers organes d'appui (4) prévus sur la première membrane et dont la surface supérieure sert de palier à l'une des deux têtes de l'articulation, le coussin et la membrane étant reçus par un corps de base (1),
**caractérisée en ce qu'**
elle comporte au-dessus de la première membrane (2), en parallèle à celle-ci, une seconde membrane (3) munie d'orifices de fixation,
les organes d'appui (4) sont ancrés dans la seconde membrane (3) et ils s'appuient latéralement les uns contre les autres et, par leur face inférieure (4e), ils s'appuient contre la première membrane (2), et
les deux membranes (2, 3) sont fixées au corps de base (1).

2. Endoprothèse d'articulation selon la revendication 1,
**caractérisée en ce que**
les organes d'appui (4) sont subdivisés en une partie supérieure (4c) et une partie de talon (4d) par des rétrécissements (4b) parallèles aux membranes (2, 3), et par leurs rétrécissements (4b), ils sont fixés dans les orifices de fixation de la seconde membrane (3).

3. Endoprothèse d'articulation selon la revendication 2,
**caractérisée en ce que**
les organes d'appui (4) ont une forme de T en coupe longitudinale.

4. Endoprothèse d'articulation selon les revendications 1,2 ou 3,
**caractérisée en ce que**
les organes d'appui (4) ont une section hexagonale.

5. Endoprothèse d'articulation selon les revendications 1 à 4,
**caractérisée en ce que**
le corps de base est réalisé sous la forme d'une cuvette (1) dont les parois latérales (la) sont prolongées jusqu'à la hauteur de la surface supérieure des organes d'appui (4a) pour soutenir latéralement les organes d'appui (4) situés à l'extérieur.

6. Endoprothèse d'articulation selon les revendications 1 à 5,
**caractérisée en ce que**
les membranes (2, 3) sont reliées solidairement au corps de base (1) de préférence par l'intermédiaire de vis (6, 7) traversant les parois (la) du corps de base (1).

7. Endoprothèse d'articulation selon les revendications 1 à 6,
**caractérisée en ce que**
les membranes (2, 3) sont en silicone et les organes d'appui (4) ainsi que la cuvette (1) sont en polyéthylène.

8. Endoprothèse d'articulation selon les revendications 1 à 7,
**caractérisée en ce que**
la cuvette (1) est remplie d'une solution de sel de cuisine ou d'une substance en forme de gel pour constituer le coussin (5), et le côté servant à recevoir la charge est fermé de manière étanche par la première membrane souple (2).

9. Endoprothèse d'articulation selon les revendications 1 à 8, selon laquelle
l'endoprothèse d'articulation est une prothèse d'articulation du genou dont la tête d'articulation est reliée au tibia ou au fémur.
